# EUROPEAN PATENT APPLICATION

(11) **EP 4 840 477 A1**
(43) Date of publication of application: **18.11.2026**
(21) Application number: 25382481.7
(22) Date of filing: 12.05.2025
(51) Int. Cl.: A61L 27/18, A61L 27/38, A61L 27/52

(54) **COMBINATIONAL DEVICE COMPRISING MESENCHYMAL STEM CELLS (MSCS) TO TREAT OCULAR PATHOLOGIES**

(71) Applicant: Instituto de Salud Carlos III, 28029 Madrid (ES)
(72) Inventor: GARCÍA RODRÍGUEZ, Patricia, 28220 Majadahonda (ES); RODRÍGUEZ MILLA, Miguel Ángel, 28220 Majadahonda (ES); GARCÍA-CASTRO, Javier, 28220 Majadahonda (ES); MARIÑAS PARDO, Luis, 28220 Majadahonda (ES)
(74) Representative: Hoffmann Eitle

(57) **Abstract**

The present invention refers to a combinational device for the delivery of an advanced therapy, that employs a silicone hydrogel (Si-Hy) scaffold for the support of mesenchymal progenitor cells (MSCs), in particular for the treatment of human and/or veterinary pathologies, and specifically ocular and skin pathologies.

## Description

### TECHNICAL FIELD

The present invention refers to the biomedical field, more particularly to a combinational device for the delivery of an advanced therapy, that employs a silicone hydrogel (Si-Hy) scaffold for the support of mesenchymal progenitor cells (MSCs), preferably for the treatment of human and/or veterinary pathologies, and specifically ocular and skin pathologies.

### BACKGROUND ART

### Corneal epithelium damage

The cornea is the first layer of the eye, and it plays a crucial role in the vision process as the interface contact with the outside world (1) and consists of multilayers of cellular (epithelium, stroma, and endothelium) and acellular (Bowman's and Descemet's membranes) components.

The corneal epithelium is a multilayered non-keratinized squamous layer formed by the renewal of limbal stem cells and their migration to the center of the cornea. They have microvilli on their surface to anchor the tear film and have, in addition, a large number of tight intercellular junctions. Limbal stem cells (LSCs) located at the corneo-conjunctival junction are crucial for corneal surface repair. In mild damage, LSCs activate, proliferate, and migrate to aid in tissue regeneration. However, if the limbal region is affected, corneal epithelium regeneration fails, leading to discomfort, vision loss, and inflammation with potential permanent scarring (2). Moreover, the cornea is the most densely innervated tissue in the body and the corneal nerves are responsible for temperature, touch, pain sensations, blink reflexes, tear production, and wound healing. The nerve supply to the cornea is derived from the ophthalmic division of the trigeminal nerve (3).

A variety of eye diseases may lead to corneal epithelium damage. One of the most devastating ocular surface disorders are limbal stem cell deficiency (LSCD) and neurotrophic keratopathy (KN).

In cases of Limbal Stem Cell Deficiency (LSCD), the pathology represents an advanced stage of morbidity resulting from a significant reduction and/or dysfunction of limbal epithelial stem cells, which play a vital role in the continuous renewal of the corneal epithelium. This condition can be triggered by a wide array of ocular surface disorders, including chemical, thermal, or mechanical injuries, immune-mediated disorders, congenital anomalies, complications from multiple ocular surgeries, or infections (4). Consequently, this leads to impaired regeneration of the ocular surface, ultimately resulting in persistent epithelial defects, secondary infections, neovascularization, and chronic inflammation. Any of these complications can contribute to corneal opacity, vision loss, and chronic pain. LSCD (ORPHA:171673) is classified as a rare disease, with an estimated prevalence of one to five individuals per 10,000 in Europe.

KN is a degenerative disease of the corneal epithelium resulting from impaired corneal innervation. A reduction in corneal sensitivity or complete corneal anesthesia is the hallmark of this disease and is responsible for producing epithelial keratopathy, ulceration and perforation (5). Although numerous ocular and systemic diseases may result in neurotrophic keratopathy, there is one common insult: a lesion of the trigeminal nerve (cranial nerve V) or its branches. The cornea has a high density of nerve endings from the long posterior ciliary nerves and is 100 times more sensitive than the conjunctiva. In the presence of neuronal destruction, epithelial cells swell, lose microvilli and produce abnormal basal lamina which leads to epithelial breakdown. NK (ORPHA:137596) is a rare disease with a prevalence of less than 5 persons in 10,000 in Europe. On average, 6 % of cases of herpetic keratitis may progress to this disease, with a peak of 12.8 % of cases of herpes zoster keratitis.

### Cell Therapy in corneal damage.

Cell therapy involves using cells as a therapeutic material and can be obtained from a donor (allogeneic use) or isolated from the patient (autologous use) and can be manipulated outside of the body to increase their number or therapeutic efficacy. The first advanced therapy medicinal product (ATMP) approved in Europe by the European Medicines Agency was Holoclar (EMEA/H/C/002450) that employs autologous LSCs (6). The procedure covers several steps: first, a biopsy of 2 × 2 mm of healthy corneal limbus is removed from patients' eye. The tissue is transported to the pharmaceutical company. Cells then are cultured for several days, a ready-for-transplant graft is made, seeding them on fibrin hydrogel biomaterial and cultured upon total coverage. This procedure has several limitations. In unilateral cases, autologous transplantation of cultured limbal epithelial stem cells can be performed using a small biopsy from the healthy contralateral eye. However, this requires additional surgery and excludes the majority of bilateral cases. Additionally, extracting the biopsy from the less affected eye could worsen its condition by removing part of the limbus. The use of allogeneic cells could solve some of these problems, but new issues would arise such as the use of immunosuppressive therapy.

In recent years, MSCs have gained significant relevance in the field of cellular therapy and regenerative medicine due to their high therapeutic potential. MSCs are non-immunogenic cells, so allogeneic transplants of MSCs do not require recipient immunosuppression. These cells are able to differentiate into bone, adipose and cartilage tissue cells and can be found in bone marrow, adipose tissue, Wharton's jelly of the umbilical cord, dental ligaments, amniotic membrane, and other tissues (7,8). One major research area is corneal epitheliopathies, where MSCs can promote corneal epithelial repair and prevent visually devastating sequelae of non-healing wounds (9). The therapeutic potential of LSCs and MSCs has also been compared with similar therapeutic effects (10), making them an attractive clinical alternative in ocular diseases.

The classical cell replacement therapy uses MSCs to functionally reconstitute multiple cell types. In congenital cornea diseases, the therapeutic effect of MSCs is at least partially attributed to either the supplementation or substitution of corneal cells. In different mice models (11,12), the introduced MSC assumed keratocytes morphology and function. The transplanted cells laid down the essential structural components in cornea stroma such as, lumican and keratocan, which helped to re-organized the collagen fibrils leading to normal corneal thickness and transparency.

### MSCs-Derived Exosomes for Cell-Free Therapy

An increasing body of evidence indicates that MSCs may exert their therapeutic effects largely secreting several signaling molecules, such as neurotrophic factors, growth factors or cytokines, which can diffuse in the local tissue environment and interact with the surrounding cells (13). Growth factors, cytokines, chemokines, extracellular matrix components, mRNAs, and regulatory miRNAs were all found to be functional molecules secreted by MSCs in various therapeutic applications (14). Some of these factors are enclosed into extracellular vesicles (EVs - which include exosomes with an average diameter of ~100 nanometres) and are involved in cell-to-cell communication and cell signaling. Exosome-based medicines are cell-free advanced therapies that circumvent some of the tricky scientific problems of cell therapy (15), such as possible abnormal differentiation or immune rejection in allogeneic scenarios, reduces the dose required to obtain therapeutic effect (leading to a lower cost per dose), prevents thrombus formation and therefore increases the safety profile of the medicine. EVs have been recognized as promising candidates for developing novel therapeutics for a wide range of pathologies, including ocular disorders (16,17).

It has been reported that hMSC-derived exosomes can be taken up by corneal epithelium in vivo (after linear scratches). The distribution of exosomes throughout the mouse cornea can be detected even 4 h after topical treatment on corneal surface (18, 19). This outcome, coupled with our present discoveries, indicates that exosomes might represent a promising avenue for treating corneal defects. However, topical treatment necessitates repeated applications within short intervals due to their brief half-life (20).

Moreover, there is currently no regulatory guidance or extracellular vesicle-based therapy approved by the EMA or the FDA, so there is still a high degree of uncertainty given the difficulties of regulatory agencies in dealing with emerging fields (21).

### Scaffolds for MSC-based Ocular Advanced Therapies.

Due to the superficial nature of the corneal environment, the retention rate of stem cells during corneal repair is suboptimal, which limits the direct application of stem cells in mending epithelial corneal injuries. To improve the retention rate of stem cells on the damaged ocular surface and for corneal epithelial regeneration, many researches have focused on the development of engineered scaffolds with or without cells (22), such as decellularized scaffolds, hydrogel scaffolds, cell sheet scaffolds, etc (23). However, there is still a need to develop cell-based therapy devices employing Mesenchymal Stem Cells (MSCs) to treat ocular pathologies such as DED and LSCD, with the aim, among others, of inducing corneal epithelial regeneration, decreasing inflammation, maintaining the functionality of ocular surface tissues (cornea, limbus, and conjunctiva), including tear-secreting glands, and thus restricting disease progression.

### Bibliography.

1 -Gehring WJ. Rev Dev Biol (2014) 3(1):1-40.
2 - Chen JJ, Tseng SC. Invest Ophthalmol Vis Sci (1990) 31(7):1301-14
3 - Pham TL & Bazan HEP. J Lipid Re. (2021) 62:100033.
4 - NaPier E et al. Ann Med (2022) 54(1):666-673
5 - Ghareeb AE et al. Ophthalmol Ther (2020) 9(4):809-831.
6 - European Medicines Agency. Holoclar: EPAR - Summary for the public (2015)
7 - Pittenger MF et al. NPJ Regen Med (2019) 4:22
8- Wu X et al. Stem Cell Res Ther (2020) 11:345
9 - Soleimani M et al. Expert Opin Biol Ther (2023) 23(6):509-525.
10 - Holan V et al. Stem Cells Transl Med (2015) 4(9):1052-63.
11- Liu H et al. PLoS One (2010) 5(5):e10707
12 - Soleimani M et al. Cells. (2023) 12(23):2744
13 - Venkatakrishnan J et al. Ocul Surf (2022) 26:255-267
14 - Han Y et al. Signal Transduct Target Ther (2022) Mar 21;7(1):92.
15 - Moghadasi S et al. J Transl Med (2021) 12;19(1):302
16- Wang M et al. Biomed Pharmacother (2023) 165:115206
17 - Yi W et al. Proc Natl Acad Sci U S A. (2024) 121(6):e2305947121
18 - Samaeekia R et al. Invest Ophthalmol Vis Sci (2018) 59(12):5194-5200
19 - Zhao W et al. Inflamm Regen (2023) 26;43(1):6
20 - Massoumi H et al. Int J Mol Sci (2023) 24(10):9006
21 - Asadpour A, et al. Stem Cell Res Ther (2023) 14(1):111
22 - Beetler DJ et al. Mol Aspects Med (2023) 91:101138
23 - Liu Y & Hong J. Cornea (2024). Epub ahead of print. PMID: 38289027.
24 - Koizumi et al. Ophthalmology (2001) 108(9):1569-74
25 - Alió Del Barrio JL, et al. Cells (2022) 11(16):2549

### BRIEF DESCRIPTION OF THE FIGURES

**Figure 1****.** Comparison of MSC growth in silicone hydrogels with phase contrast light microscopy after 2 days of culture. Adhesion and positive growth corresponds to a lens composed of Lotrafilcon B that allows growth. The second image with absence of adhesion and growth corresponds to an Asmofilcon A lens.
**Figure 2****.** MSC-hTERT-GFP on Lotrafilcon B lens with plasma gas surface treatment in confocal microscopy after 2 days of culture. A and B) Full surface of the contact lens showing the cells growing on its surface B) is the image taken with the 10x GFP channel. C) Image that corresponds to the use of the DAPI, SiR-Actin Kit and GFP channels.
**Figure 3****.** Electron microscopy image showing MSC-hTERT-GFP on Lotrafilcon B lens with plasma gas surface treatment. The strong union of the cell through a developed cortical cytoskeleton is observed. The absence of degradation in the contact lens is also observed.
**Figure 4****:** Composition showing the absence of adhesion and growth in other silicone hydrogels.

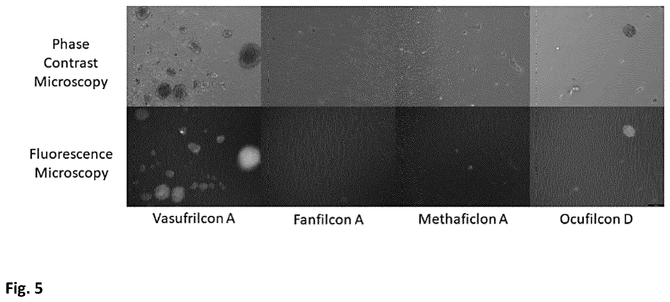

**Figure 5****:** Composition showing the absence of adhesion and growth in other silicone hydrogels.

### DESCRIPTION OF EMBODIMENTS OF THE INVENTION THE INVENTION

### Silicone Hydrogel Scaffold for Stem Cell-Based Therapy in Ocular Disorders

The product of the present invention is a combinational device for the delivery of an advanced therapy, that employs a silicone hydrogel (Si-Hy) scaffold for the support of mesenchymal progenitor cells (MSCs), in particular for the treatment of human and/or veterinary pathologies, and specifically ocular and skin pathologies.

The silicone hydrogel scaffold is based on N, N dimethyl acrylamide (DMA) and trimethylsiloxy silane (TRIS) (United States Adopted Name Council - USAN: LOTRAFILCON). The scaffold must be polymerized, moulded or carved in different shapes and sizes, but mainly in two dimensions. This Si-Hy is solid and inert, not injectable, biodegradable nor bioabsorbable.

The mesenchymal progenitor cells could be derived from adipose tissue, bone marrow or Wharton jelly. Will be seeded and grown in at least one side of the 2D scaffold surface, not encapsulated into the Si-Hy or embedded to form spheroids (3D). The side where the cells are attached and grown must suffer a surface plasma treatment prior to cell attachment to increase the wettability of the scaffold. No additional peptide or polysaccharide coating is preferably employed (i.e. hyaluronic acid, collagen, fibrin, alginate...).

The MSCs can be obtained from a primary culture or be an immortalized cell line. The combinational device can be employed in an autologous, allogeneic or xenogeneic scenario. The scaffold allows for strong cell adhesion, and progenitors will remain attached throughout the *in vivo* use of the device. The ultimate goal is to bring MSCs into contact with an injured area where the secretome of the cells can reach the host tissue and promote healing, but not the implantation of the cells themselves. Thus, after the indicated period of use, the device will be removed with the cells still attached. By eliminating the risk that cell implantation could pose, it opens up the possibility of using the cells allogeneically or xenogeneically, limiting or eliminating the risk of rejection.

It is further noted, that the Si-Hy is not a cohesive group and its development as a biomedical material has been intensively pursued especially in the field of ophthalmology, where it is used for the manufacture of contact lenses. In contrast to hydrogels, Si-Hy rely on silicon rather than water to transport oxygen through the device.

When these polymers are employed in the manufacture of soft contact lenses, are grouped by FDA into different groups depending on their water and ionic content. The United States Adopted Names (USAN) Council establishes logical nomenclature classifications based on pharmacological and/or chemical relationships and is responsible for selecting simple, informative and unique non-proprietary (generic) drug names.

The polymer employed in this invention belongs to the **FDA Group I (<50% WC <0.2% ionic content)** and has the USAN LOTRAFILCON (A and/or B).

The first-generation silicone hydrogels, such as lotrafilcon, use the TRIS molecule that has three methacrylated trimethyl silicone groups stemming from a primary silicone group. Lotrafilcon concentrates on siloxy macromers in addition to a TRIS-based backbone.

Lotrafilcon took advantage of TRIS and other hydrophilic monomers to phase independently, producing a material that has two distinct phases: a water and gel phase that the manufacturer calls co-continuous phases. This allows for high oxygen permeability, as well as high water and sodium permeability, important for the viability of the MSCs cultured on the inner side of the 2d matrix once they are implanted.

It is additionally noted that the Lotrafilcon A/B must suffer a surface treatment to modify the surface. Surface can be modified using either corona discharge or gas-plasma. These processes generate highly energetic oxygen ions which graft onto the surface polymer chains so that the surface becomes hydrophilic and negatively charged when medium is added. The more oxygen that is incorporated on to the surface the more hydrophilic it becomes and the better it is for cell attachment and spreading.

Originally this surface treatment was developed for contact lenses to buffer the silicone from the ocular surface and the tear layer, but this process adds properties to the surface that makes mesenchymal stem cells to attach to the surface. The second and third lenses generations aren't surface treated. They use polyvinyl pyrrolidone (PVP) as an internal wetting agent. PVP is a long chain, high molecular weight molecule that sequesters the silicone within the core of the lens. In other words, it provides a hydrophilic layer on the surface of the hydrogel.

In the past, plasma-treated silicone hydrogel scaffolds have been used for the culture and transfer of corneal epithelial cells (Brown et al., 2014) and limbal stem cells (Bobba et al. 2016) for the treatment of ocular pathologies or keratinocytes for the treatment of dermatological pathologies (Haddow et al. 2003; Moustafa et al. 2004).

However, there are huge differences with the present invention, both in the type of hydrogel used, the cell type (corneal epithelial cells, limbal stem cells and keratinocytes vs. MSCs), as well as the mode of action. The aim of these cited works was specifically the transfer of the cell cultures in order to be implanted in the recipient. However, the aim of the present invention is that the cells remain tightly bound to the scaffold and exert their action through MSCs secreted mediators. In the present invention, after the healing effect the scaffold is removed and with it, the cells growing on its surface. Thus, eliminating the risk that cellular implantation could pose, and opening up the possibility of using allogeneic and xenogeneic treatments with lower or not risk of rejection.

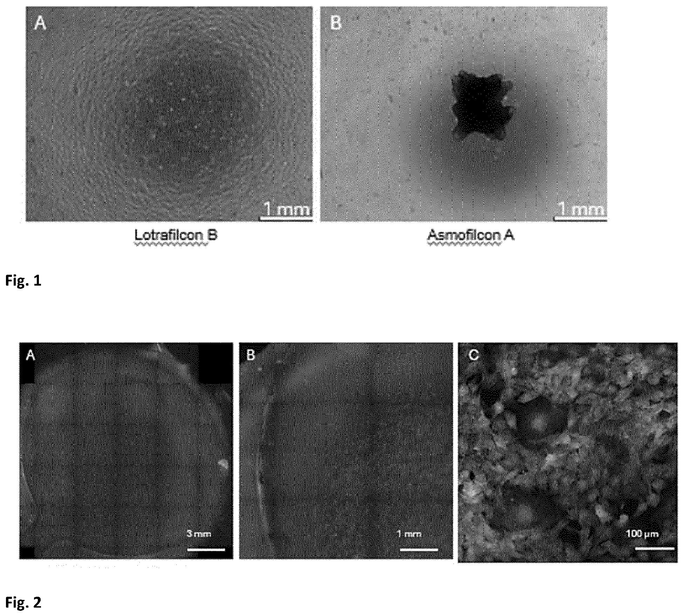

Consequently, the present invention describes the development of a new therapeutic strategy based on a combinational device as cell-based therapy employing Mesenchymal Stem Cells (MSCs) to treat ocular pathologies such as DED and LSCD, with the aim, among others, of inducing corneal epithelial regeneration, decreasing inflammation, maintaining the functionality of ocular surface tissues (cornea, limbus, and conjunctiva), including tear-secreting glands, and thus restricting disease progression. This combinational device combines the positive aspects of both MSCs and EV treatments, while avoiding their respective drawbacks. MSCs are cultured on the surface of scaffolds, and once applied onto the corneal surface, the secretion of EVs will exert the expected therapeutic effects (See the conceptual design of the device in Figure 1A).

In this invention, we employ a specific scaffold that allows for strong cell adhesion, and progenitors will remain attached throughout the in vivo use of the device. The ultimate goal is to bring MSCs into contact with an injured area where the secretome of the cells can reach the host tissue and promote healing, but not the implantation of the cells themselves. Thus, after the indicated period of use, the device will be removed with the cells still attached. By eliminating the risk that cell implantation could pose, it opens up the possibility of using the cells allogeneically or xenogeneically, limiting or eliminating the risk of rejection.

Therefore, a first aspect of the invention refers to a biomedical scaffold, comprising:
(a) a porous substrate formed of a biocompatible material consisting of the silicone-hydrogel polymer known as LOTRAFILCON A and/or LOTRAFILCON B; and
(b) a plasma-treated surface layer on at least a portion of the porous substrate, preferably on only one side of the porous substrate,
wherein the plasma treatment is configured to increased hydrophilicity relative to an untreated scaffold formed of the same biocompatible material.

Lotrafilcon A and Lotrafilcon B are commercial, second-generation silicone-hydrogel materials used in high-DK contact lenses. They are formed by free-radical cross-linking of siloxane-containing macromers (e.g., tris-(trimethyl-siloxy)-silyl-propyl methacrylate) with hydrophilic monomers (e.g., N,N-dimethylacrylamide) plus fluorinated comonomers. Phase separation of the hydrophobic siloxane blocks during cure creates a three-dimensional network of nanometer-scale, interconnected voids (< 50 nm) that provide gas channels-hence "porous substrate." The bulk water content is 24 wt % (Lotrafilcon A) or 33 wt % (Lotrafilcon B), while the surface is inherently hydrophobic and must be modified to achieve on-eye wettability.

On another note, methods are known in the art for generating a plasma-modified surface layer on at least a portion of a soft contact lens formed from a porous silicone-hydrogel polymer, such as Lotrafilcon A or Lotrafilcon B. These known methods typically involve placing the lens in a low-pressure plasma chamber and exposing the lens surface to an ionized gas, for example an oxidizing or inert plasma, under conditions designed to remove loosely bound oligomers and introduce polar functional groups. In certain variations, one or more hydrophilic monomers are plasma-polymerized or grafted onto the activated substrate, forming a conformal coating that preserves the underlying gas-permeable network and enhances the wettability of the final lens surface. Such methods may be applied either to the entire lens surface or only to selected regions, for instance by use of masks or controlled electrode geometry, and generally do not require extensive modification of standard vacuum or plasma-processing equipment.

The described process yields Lotrafilcon A or B contact lenses with a durable, nanometric plasma-modified surface that dramatically enhances wettability and deposit resistance while preserving the porous silicone-hydrogel's high oxygen permeability.

In an embodiment of the first aspect of the invention, the biomedical scaffold is configured for use, moulded or carved to treat human and/or veterinary ocular or skin pathologies.

In another embodiment of the first aspect of the invention, the biomedical scaffold is a 2D scaffold surface plasma-treated surface configured for use, moulded or carved as ocular contact lenses.

A "2D scaffold surface" configured for use in ocular contact lenses is generally understood in the art to refer to a substantially planar or thin-walled substrate formed from a silicone-hydrogel polymer, such as Lotrafilcon A or Lotrafilcon B, wherein a continuous pore network is present to confer high oxygen permeability. In known techniques, this scaffold surface is subjected to a plasma treatment-typically at reduced pressure and employing an ionized gas or gas mixture-so as to remove weakly bound oligomers and introduce chemical functionalities capable of improving wettability, lubricity, or resistance to deposits. The treated scaffold may thereafter be molded, carved, or otherwise shaped into a final lens configuration without substantially disturbing the thin plasma-modified layer, thereby providing a soft contact lens exhibiting enhanced on-eye comfort while retaining the intrinsic gas-transmissive properties of the underlying silicone-hydrogel matrix.

In another embodiment of the first aspect of the invention, optionally in combination with any previous or subsequent embodiments of the first aspect of the invention, the plasma treatment is performed using a gas selected from the group consisting of oxygen, nitrogen, argon, ammonia, carbon dioxide, or any combination thereof.

In another embodiment of the first aspect of the invention, optionally in combination with any previous or subsequent embodiments of the first aspect of the invention, the plasma treatment is performed at a power level in a range from about 10 W to about 500 W and for a duration in a range from about 10 seconds to about 30 minutes.

In another embodiment of the first aspect of the invention, optionally in combination with any previous or subsequent embodiments of the first aspect of the invention, the plasma treatment creates functional groups on the surface of the scaffold selected from hydroxyl (-OH), carboxyl (-COOH), amine (-NH2), carbonyl (-C=O), or combinations thereof.

In another embodiment of the first aspect of the invention, optionally in combination with any previous or subsequent embodiments of the first aspect of the invention, the plasma-treated surface layer is further coated or functionalized with one or more bioactive agents selected from the group consisting of growth factors, peptides, antibodies, nucleic acids, small molecules, or combinations thereof. More preferably, the plasma-treated surface layer is not coated or functionalized with one or more bioactive agents selected from the group consisting of growth factors, peptides, antibodies, nucleic acids, small molecules, or combinations thereof.

In another embodiment of the first aspect of the invention, optionally in combination with any previous or subsequent embodiments of the first aspect of the invention, the biomedical scaffold comprises mesenchymal progenitor cells (MSCs), preferably obtained from a primary culture or an immortalized cell line.

In another embodiment of the first aspect of the invention, optionally in combination with any previous or subsequent embodiments of the first aspect of the invention, the biomedical scaffold is a 2D scaffold surface plasma-treated surface moulded or carved as ocular contact lenses, and the 2D scaffold surface plasma-treated surface comprises MSCs seeded and grown on or onto the portion of the porous substrate treated with plasma. Preferably, wherein the MSCs are seeded and grown after the surface plasma treatment.

A second aspect of the invention refers to a method of producing a plasma-treated biomedical scaffold, the method comprising:
(a) forming a porous scaffold from a biocompatible material consisting of LOTRAFILCON; and
(b) subjecting at least a portion of the porous scaffold to a plasma treatment, preferably on only one side of the porous substrate,
wherein the plasma treatment is configured to increased hydrophilicity relative to an untreated scaffold formed of the same biocompatible material.

In an embodiment of the second aspect of the invention, the method further comprises selecting parameters for the plasma treatment from a power range of about 10 W to about 500 W, a treatment time of about 10 seconds to about 30 minutes, and a gas flow rate in a range from about 1 sccm to about 100 sccm.

In another embodiment of the second aspect of the invention, optionally in combination with any previous or subsequent embodiments of the second aspect of the invention, the method further comprises the post-plasma functionalization of the scaffold surface with a bioactive agent.

In another embodiment of the second aspect of the invention, optionally in combination with any previous or subsequent embodiments of the second aspect of the invention, the porous scaffold is a 2D scaffold surface plasma-treated surface configured for use, moulded or carved as ocular contact lenses.

In another embodiment of the second aspect of the invention, optionally in combination with any previous or subsequent embodiments of the second aspect of the invention, the biomedical scaffold comprises MSCs seeded and grown on the portion of the porous substrate treated with plasma and wherein the MSCs are seeded and grown after the surface plasma treatment.

In another embodiment of the second aspect of the invention, optionally in combination with any previous or subsequent embodiments of the second aspect of the invention, the biomedical scaffold is a 2D scaffold surface plasma-treated surface configured for use, moulded or carved as ocular contact lenses.

Finally, a third aspect of the invention refers to a biomedical scaffold as defined in any of the embodiments of the first aspect of the invention, for use in the prevention or treatment of ocular pathologies, preferably for use in the treatment of ocular pathologies such as DED and LSCD, with the aim, among others, of inducing corneal epithelial regeneration, decreasing inflammation, maintaining the functionality of ocular surface tissues (cornea, limbus, and conjunctiva), including tear-secreting glands, and thus restricting disease progression. Preferably the biomedical scaffold is the combinational device for the delivery of an advanced therapy defined herein, that employs the silicone hydrogel (Si-Hy) scaffold as the support of mesenchymal progenitor cells (MSCs).

### EXAMPLES

### MATERIALS AND METHODS

**Table 1. Classification of the lenses used.**

| ***FDA-compliant group*** | **Group I** | **Group II** | **Group IV** |
|---|---|---|---|
| | Lotrafilcon B | Alphafilcon A | Methafilcon A |
| *Polymer Employed* | Lotrafilcon A | Omafilcon A | Etafilcon A |
| | Asmofilcon A | Vasurfilcon A | Ocufilcon D |
| | Comfilcon A | Fanfilcon A | |

### METHODS

### Cell cultures:

MSC-hTERT-GFP cells are seeded in 175 cm2 flask-type culture jars with DMEM. They are incubated at 37°C in an atmosphere with 5% CO₂. The change of medium is made according to the pH; If the medium takes on a more orange and less pink color, it should be replaced, as this indicates an increase in acidity. When the cells reach approximately 80% confluence, a cell pass is performed to keep the lineage alive.

For cell passes, the medium is removed. This is followed by 3 washes with PBS 1X, followed by treatment with TrypLE 1X at 37ºC for at least 5 minutes to detach the cells from the bottle. The detached cells are collected and transferred to a Falcon tube with DMEM medium to inactivate the enzyme (TrypLE). Then, the tube is centrifuged and, if a pellet is obtained, it is re-suspended and the cells that express GFP are counted. Once the cells have been counted, 5·10⁵ to 10⁶ MSC-hTERT-GFP cells are seeded into another culture flask and the remaining medium is added to a volume of 25 to 35 mL in the container, depending on how long the cells will be unsupervised or without changing media.

### Cell cultures on contact lenses:

Regardless of the type of lens used, the same procedure is always performed. In a 24-well plate, 1 to 1.5 mL of DMEM medium is added to each well. Once the medium is added, a contact lens is placed in each well under sterile conditions. With great caution, approximately 10⁴ MSC-hTERT-GFP cells are seeded in the most concave part of the contact lens. After the cells are seeded, the plate is left static for 5 to 10 minutes, to ensure the correct adhesion of the cell to the lens. They are then incubated at 37ºC with an atmosphere of 5% CO2. The first 2-3 days, the medium is not changed.

Subsequently, the medium is changed daily, without completely removing the old medium.

### Cell staining for confocal microscopy:

For the fixation of MSC-hTERT-GFP cells grown in contact lenses, the following procedure is followed: the MSCs are fixed to the surface of the lens with 4% PFA and then washed three times with PBS 1X. Since the cells express GFP, they are stained with DAPI and the SiR-actin kit, so that they can be perceived under the confocal microscope. First, DAPI is applied after fixation. The SiR-actin kit is then used according to the manufacturer's instructions. Once this process is completed, the plate is covered with aluminum foil and stored in the refrigerator at 4ºC until visualization under the confocal microscope. Once taken to the microscope, with a sterile blade, cuts will be made in the outermost part of the lens so that it loses its concavity and acquires a flat structure. This makes it easier to take sharper and better quality images.

**Fixation and preparation of MSC-hTERT-GFP in contact lens for electron microscopy:**
The plate presenting the lenses is taken with MSC-hTERT-GFP. Once a desired cell density is reached in the center of the lens (around 70%), fixation is carried out. To carry out this process, the protocol described in Quereda et al., 2017 (69) is followed. After removing the DMEM from the well, add the fixative (2% PFA, 1% GA (glutaraldheide) in PHEM buffer at a pH of 7.2), seal the plate with Parafilm and leave at 4ºC.

### RESULTS

By means of phase contrast light microscopy (fig 1.), the growth of these MSCs in the hydrogels can be verified. In Lotrafilcon A and B lenses with a surface treatment of 25nm using plasma gas, it is observed that the cells have expanded and colonized the lens, while in lenses with other compositions and surface treatments, an agglomeration of MSCs is observed, showing the absence of adhesion and proliferation of these cells.

The formation of clumps is due to the lack of initial adhesion combined with the concave structure of the contact lenses (which favors the accumulation of cells in the deeper region of the lens).

With the stains used for confocal microscopy, it is observed that MSC-hTERT-GFP at the time of fixation are alive in contact lenses, since they express GFP (fig. 2. A and B) and monolayer growth is confirmed (fig 2.C). With this evidence (Figs. 1 and 2.) we can conclude that MSC cells grow successfully on those formed by Lotrafilcon B with surface treatment by plasma gas and that they also do so in monolayer.

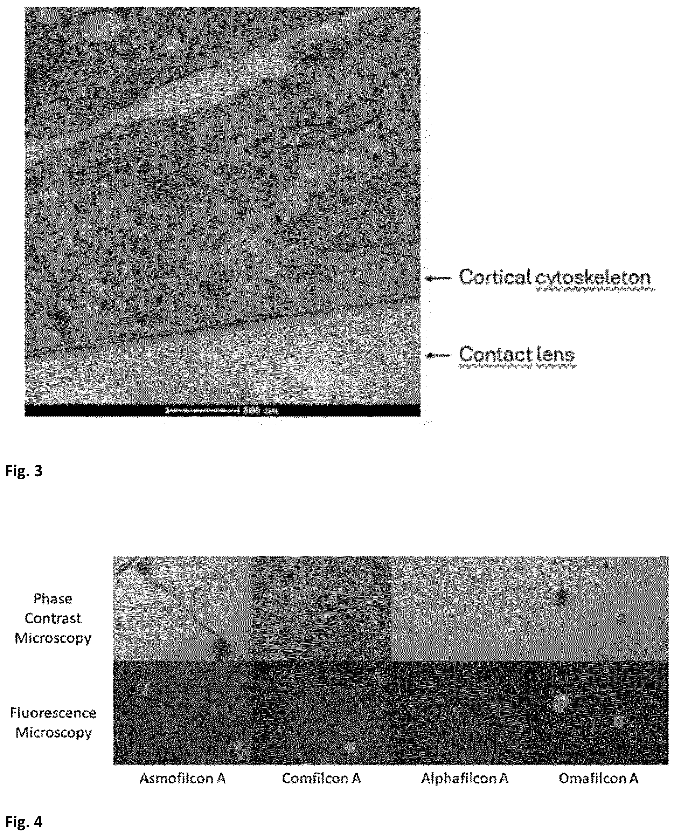

Electron microscopy shows that most cells develop a powerful cortical cytoskeleton in the hydrogel-binding area (Fig. 3), suggesting strong adhesion to the hydrogel. The image also shows that cell adhesion and growth do not cause degradation.

## Claims

1. A biomedical scaffold, comprising:
(a) a porous substrate formed of a biocompatible material consisting of the silicone-hydrogel polymer known as LOTRAFILCON A and/or LOTRAFILCON B; and
(b) a plasma-treated surface layer on at least a portion of the porous substrate, preferably on only one side of the porous substrate,
wherein the plasma treatment is configured to increased hydrophilicity relative to an untreated scaffold formed of the same biocompatible material.

2. The biomedical scaffold of claim 1, wherein the biomedical scaffold is configured for use, moulded or carved to treat human and/or veterinary ocular or skin pathologies.

3. The biomedical scaffold of claim 1 or 2, wherein the biomedical scaffold is a 2D scaffold surface plasma-treated surface configured for use, moulded or carved as ocular contact lenses.

4. The biomedical scaffold of claim 1, wherein the plasma treatment is performed using a gas selected from the group consisting of oxygen, nitrogen, argon, ammonia, carbon dioxide, or any combination thereof.

5. The biomedical scaffold according to any one of claims 1 to 4, wherein the plasma treatment is performed at a power level in a range from about 10 W to about 500 W and for a duration in a range from about 10 seconds to about 30 minutes.

6. The biomedical scaffold according to any one of claims 1 to 5, wherein the plasma treatment creates functional groups on the surface of the scaffold selected from hydroxyl (-OH), carboxyl (-COOH), amine (-NH2), carbonyl (-C=O), or combinations thereof.

7. The biomedical scaffold according to any one of claims 1 to 6, wherein the plasma-treated surface layer is further coated or functionalized with one or more bioactive agents selected from the group consisting of growth factors, peptides, antibodies, nucleic acids, small molecules, or combinations thereof.

8. The biomedical scaffold according to any one of claims 1 to 7, wherein the biomedical scaffold comprises mesenchymal progenitor cells (MSCs), preferably obtained from a primary culture or an immortalized cell line.

9. The biomedical scaffold according to claim 8, wherein the biomedical scaffold is a 2D scaffold surface plasma-treated surface moulded or carved as ocular contact lenses, and the 2D scaffold surface plasma-treated surface comprises MSCs seeded and grown on the portion of the porous substrate treated with plasma.

10. The biomedical scaffold according to claim 9, wherein the MSCs are seeded and grown after the surface plasma treatment.

11. A method of producing a plasma-treated biomedical scaffold, the method comprising:
(a) forming a porous scaffold from a biocompatible material consisting of LOTRAFILCON; and
(b) subjecting at least a portion of the porous scaffold to a plasma treatment, preferably on only one side of the porous substrate,
wherein the plasma treatment is configured to increased hydrophilicity relative to an untreated scaffold formed of the same biocompatible material.

12. The method of claim 11, further comprising selecting parameters for the plasma treatment from a power range of about 10 W to about 500 W, a treatment time of about 10 seconds to about 30 minutes, and a gas flow rate in a range from about 1 sccm to about 100 sccm.

13. The method of claim 11 or 12, further comprising post-plasma functionalization of the scaffold surface with a bioactive agent.

14. The method according to any one of claims 11 to 13, wherein the porous scaffold is a 2D scaffold surface plasma-treated surface configured for use, moulded or carved as ocular contact lenses.

15. The method according to any one of claims 11 to 14, wherein the biomedical scaffold comprises MSCs seeded and grown on the portion of the porous substrate treated with plasma and wherein the MSCs are seeded and grown after the surface plasma treatment.

16. The method according to claim 15, wherein the biomedical scaffold is a 2D scaffold surface plasma-treated surface configured for use, moulded or carved as ocular contact lenses.

17. A biomedical scaffold as defined in any of claims 8 to 10, for use in the prevention or treatment of ocular pathologies.
